# FASCICULE DE BREVET EUROPEEN

(11) **EP 3 803 323 B1**
(45) Date de publication et mention de la délivrance du brevet: **23.03.2022**
(21) Numéro de dépôt: 19730689.7
(22) Date de dépôt: 21.05.2019
(51) Int. Cl.: G01N 1/26

(54) **STATION ET PROCÉDÉ DE MESURE DE LA CONTAMINATION MOLÉCULAIRE VÉHICULÉE PAR L'AIR**
STATION UND VERFAHREN ZUR MESSUNG DER MOLEKULAREN KONTAMINATION IN DER LUFT
STATION AND METHOD FOR MESURING AIRBORNE MOLECULAR CONTAMINATION

(30) Priorité: 28.05.2018 FR 1800562
(43) Date de publication de la demande: 14.04.2021
(73) Titulaire: Pfeiffer Vacuum, 74000 Annecy (FR)
(72) Inventeur: BOUNOUAR, Julien, 74000 Annecy (FR); LE BARILLEC, Olivier, 74370 Annecy (FR)
(74) Mandataire: Croonenbroek, Thomas Jakob
(86) Numéro de dépôt international: PCT/EP2019/063027
(87) Numéro de publication internationale: WO 2019/228844

(56) Documents cités:
- WO-A1-01/79809
- FR-A1- 2 795 517
- KR-A- 20110 026 918
- US-A- 3 043 145

## Description

La présente invention se rapporte à une station de mesure de la contamination moléculaire véhiculée par l'air, destinée en particulier à la surveillance des concentrations en contamination moléculaire dans l'atmosphère des salles blanches, telles que les salles blanches d'usines de fabrication de semi-conducteurs. La présente invention se rapporte également à un procédé de mesure de la contamination moléculaire véhiculée par l'air au moyen d'une telle station.

Dans l'industrie de fabrication de semi-conducteurs, les substrats, tels que les plaquettes de semi-conducteurs (ou « wafer » en anglais) ou les photomasques, doivent être protégés de la contamination moléculaire véhiculée par l'air (ou AMC pour « Airbone Molecular Contamination » en anglais) afin d'éviter que celle-ci n'endommage les puces ou circuits électroniques des substrats. Pour cela, les substrats sont contenus dans des boîtes de transport et de stockage atmosphérique, permettant de transporter les substrats d'un équipement à l'autre ou de les stocker entre deux étapes de fabrication. Par ailleurs, les boites de transport et les équipements sont agencés à l'intérieur de salles blanches dans lesquelles le niveau de particules est minimisé et la température, l'humidité et la pression sont maintenus à des niveaux précis.

Dans la salle blanche, les espèces gazeuses véhiculées par l'air peuvent avoir différentes sources et différentes natures, on trouve par exemple des acides, des bases, des éléments condensables, des éléments dopants. Ces molécules peuvent provenir de l'air intérieur de l'usine de fabrication de semi-conducteurs ou peuvent être relâchés notamment par les plaquettes semi-conductrices ayant subi des opérations préalables de fabrication.

Des analyseurs de gaz présents dans les salles blanches permettent d'évaluer la concentration des espèces gazeuses véhiculées par l'air en temps réel, notamment celle de l'humidité et de quelques acides. Les concentrations mesurées sont parfois très faibles, telles que de l'ordre du ppm ou du ppb. Ces analyseurs de gaz mesurant l'atmosphère gazeuse les environnants, il est donc nécessaire de prévoir un analyseur de gaz dans chaque zone à tester de la salle blanche.

Il existe un besoin d'augmenter le nombre d'espèces gazeuses mesurées et le nombre de zones de test afin de réduire les risques de contamination des substrats. Cependant, la multiplication des analyseurs par zone et la multiplication de ces zones à tester rend cette solution rapidement très couteuse.

Pour réduire les coûts, on a proposé une unité de mesure regroupant différents analyseurs. L'unité est munie de plusieurs ports d'entrée adressant chacun une zone de test particulière de la salle blanche. Les salles blanches pouvant atteindre des dimensions importantes et le nombre de zones de test étant lui aussi en augmentation, il s'avère nécessaire d'utiliser un nombre conséquent de lignes de prélèvement, les longueurs de ces lignes atteignant le plus souvent plusieurs dizaines de mètres. Ce long parcours du gaz pour atteindre la cellule de mesure de l'analyseur prend du temps, ce qui implique un retard dans l'information. Il faut en effet « remplacer » tout le volume contenu dans la ligne de prélèvement par le gaz à mesurer au moins une fois, ce gaz pouvant en outre facilement se coller aux parois de la ligne par adsorption, en particulier pour les espèces gazeuses à mesurer dites polaires. Il peut alors être difficile d'obtenir une mesure réellement représentative de la concentration des espèces gazeuses dans la zone de test à moins d'attendre un temps très long à chaque changement de zone de test.

Une solution consiste à aspirer simultanément dans toutes les lignes de prélèvement. Toutes les lignes de prélèvement sont ainsi conditionnées au moyen d'une évacuation commune, une mesure de la concentration du gaz étant réalisée dans une seule ligne à la fois. Le gaz est ainsi constamment aspiré dans toutes les lignes de prélèvement, ce qui permet notamment un bon dégazage des lignes de prélèvement. De tels systèmes connus sont divulgués par exemple dans les documents KR20110026918 A et US3043145 A.

Le nombre de lignes à prélever simultanément étant important, cette solution engendre toutefois une forte réduction du débit de pompage dans chacune des lignes. Le temps de réponse dans la ligne de prélèvement pour laquelle une mesure est réalisée est alors allongé par rapport à une solution où la totalité du débit de pompage est consacré au prélèvement de la seule ligne à mesurer. Par ailleurs, les lignes de prélèvement n'ont pas toutes la même longueur. Certaines lignes peuvent être très courtes et d'autres très longues. Les débits de pompage peuvent donc présenter de fortes disparités entre les lignes. Des cas peuvent même se présenter où la différence de longueur entre deux lignes est tellement grande qu'un débit de pompage correct dans une ligne équivaut à un débit de pompage quasiment nul dans une autre.

Une solution pourrait être d'augmenter la capacité de pompage du prélèvement simultané. Cela engendre cependant un coût relativement important.

Un des buts de la présente invention est de proposer une station de mesure qui résolve au moins partiellement les inconvénients précités.

A cet effet, l'invention a pour objet une station de mesure de la contamination moléculaire véhiculée par l'air comportant au moins un analyseur de gaz et une pompe de conditionnement caractérisée en ce qu'elle comporte en outre une unité de séquençage comportant un programme de séquençage définissant un ordre des mesures à réaliser pour au moins deux lignes de prélèvement, l'unité de séquençage étant configurée pour piloter la mise en communication d'une ligne de prélèvement à mesurer, par exemple d'une seule et unique ligne de prélèvement, dont la mesure est programmée consécutivement à une ligne de prélèvement en cours de mesure, avec la pompe de conditionnement, pendant le pilotage de la mise en communication de la ligne de prélèvement en cours de mesure avec le au moins un analyseur de gaz.

La ligne de prélèvement peut ainsi être conditionnée dans des conditions de pompage optimales et rapides.

En effet, le temps de mesure est réduit car le conditionnement de la ligne de prélèvement est réalisé en temps masqué.

En outre, un maximum de pompage peut être utilisé pour le conditionnement et ce, quelque soit le nombre de lignes de prélèvement. Le nombre de lignes de prélèvement peut donc être augmenté sans que cela modifie les performances de pompage pour le conditionnement. Le gaz ainsi aspiré avant que la ligne soit mesurée permet un dégazage de la ligne de prélèvement. Les mesures peuvent alors être plus précises, en particulier dans les cas où un changement de concentration important a lieu entre deux zones à tester consécutivement et où un temps de dégazage suffisant est nécessaire pour obtenir une mesure réellement représentative de la concentration dans la zone de test.

Il est ainsi possible de mesurer la concentration de différentes espèces gazeuses véhiculées par l'air à différents endroits de la salle blanche avec un même ensemble d'analyseurs de gaz, la station de mesure adressant un port d'entrée dans chaque zone de test de la salle blanche. On limite ainsi les coûts. L'invention prévoit une station de mesure selon la revendication 1 et un procédé de mesure selon la revendication 6.

D'autres caractéristiques et avantages de l'invention ressortiront de la description suivante, donnée à titre d'exemple, sans caractère limitatif, en regard des dessins annexés sur lesquels:
La Figure 1 représente une vue schématique d'un premier exemple de réalisation d'une station de mesure de la contamination moléculaire véhiculée par l'air.
La Figure 2 représente une vue schématique d'un procédé de mesure de la contamination moléculaire véhiculée par l'air au moyen de la station de mesure de la Figure 1.
La Figure 3 représente une vue schématique d'un deuxième exemple de réalisation d'une station de mesure de la contamination moléculaire véhiculée par l'air.
La Figure 4 représente une vue schématique d'un procédé de mesure de la contamination moléculaire véhiculée par l'air au moyen de la station de mesure de la Figure 3.

Sur ces figures, les éléments identiques portent les mêmes numéros de référence.

Les réalisations suivantes sont des exemples. Bien que la description se réfère à un ou plusieurs modes de réalisation, ceci ne signifie pas nécessairement que chaque référence concerne le même mode de réalisation, ou que les caractéristiques s'appliquent seulement à un seul mode de réalisation. De simples caractéristiques de différents modes de réalisation peuvent également être combinées pour fournir d'autres réalisations.

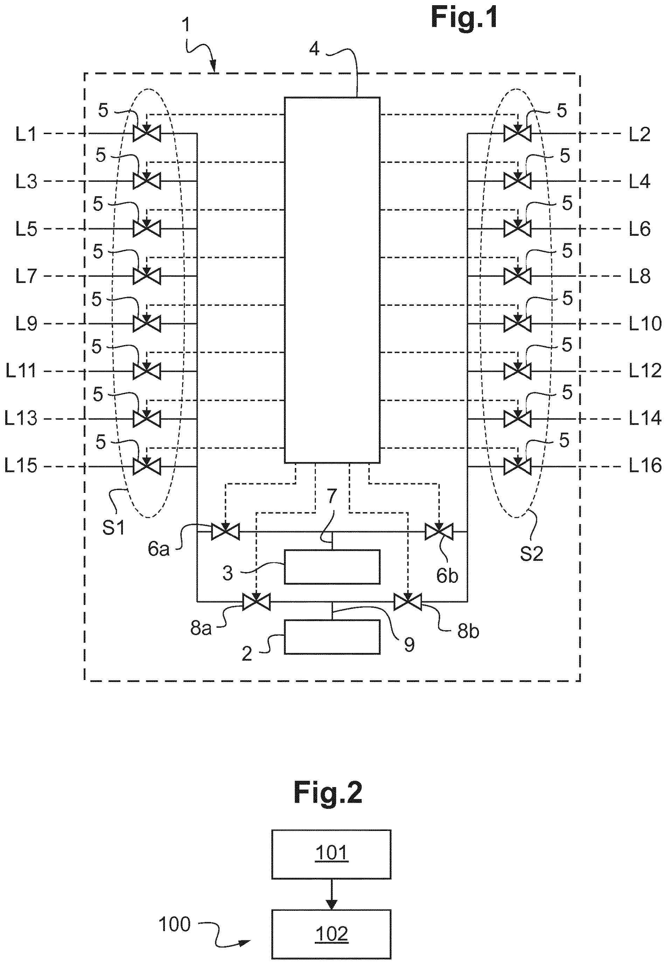

La Figure 1 montre des éléments d'une station de mesure 1 de la contamination moléculaire véhiculée par l'air, destinée en particulier à la surveillance des concentrations en contamination moléculaire dans l'atmosphère des salles blanches, telles que les salles blanches d'usines de fabrication de semi-conducteurs.

La station de mesure 1 comporte au moins un analyseur de gaz 2, une pompe de conditionnement 3 et une unité de séquençage 4.

Selon un exemple de réalisation, l'analyseur de gaz 2 comporte une petite pompe de prélèvement. L'analyseur de gaz 2 permet de mesurer la concentration d'au moins une espèce gazeuse en temps réel, c'est-à-dire avec une durée de mesure inférieure à quelques secondes, voire quelques minutes, pour de faibles concentrations inférieures au ppm ou du ppb. L'espèce gazeuse mesurée est par exemple un acide, comme l'acide fluorhydrique HF ou l'acide chlorhydrique HCl ou un solvant, tel que le PGMEA (propylène glycol methyl ether). Selon un autre exemple, l'espèce gazeuse est l'ammoniaque NH₃. Un analyseur de gaz 2 peut être adapté pour la mesure d'une espèce gazeuse distincte ou d'un groupe d'espèces gazeuses distinctes.

La pompe de conditionnement 3 comporte par exemple une pompe à vide de petite capacité, telle qu'une petite pompe à vide multiétagée.

L'unité de séquençage 4 est par exemple un ordinateur.

L'unité de séquençage 4 comporte un programme de séquençage définissant un ordre des mesures à réaliser pour au moins deux lignes de prélèvement parmi des lignes de prélèvements L1- L16.

Les lignes de prélèvements L1- L16 comportent par exemple des tuyaux flexibles, réalisés dans des matériaux limitant l'adhérence des espèces gazeuses aux parois, tel qu'en perfluoroalkoxy (également appelé PFA) ou en polytétrafluoroéthylène (également appelé PTFE). Les lignes de prélèvements L1- L16 relient la station de mesure 1 à des zones de test particulières de la salle blanche. La longueur des lignes de prélèvement L1- L16 peut varier entre les différentes zones de test à rallier et peut présenter plusieurs dizaines de mètres, telle qu'une longueur comprise entre 40 et 200 mètres.

Le programme de séquençage définit l'ordre des mesures à réaliser c'est-à-dire dans quel ordre les mesures de concentrations des espèces gazeuses doivent être réalisées dans les lignes de prélèvement L1-L16 reliées à la station de mesure 1.

L'unité de séquençage 4 est en outre configurée pour piloter la mise en communication d'une ligne de prélèvement L1-L16, par exemple d'une seule et unique ligne de prélèvement, à mesurer avec la pompe de conditionnement 3 pendant qu'elle pilote la mise en communication de la ligne de prélèvement L1-L16 en cours de mesure avec le au moins un analyseur de gaz 2.

On distingue ainsi une ligne de prélèvement « en cours de mesure » d'une ligne de prélèvement « à mesurer ». La ligne de prélèvement « à mesurer » parmi les lignes de prélèvement L1-L16 est celle dont la mesure est programmée consécutivement à la ligne de prélèvement L1-L16 en cours de mesure.

Pour cela, la station de mesure 1 comporte :
- une électrovanne de prélèvement 5 pilotable par l'unité de séquençage 4 sur chaque ligne de prélèvement L1-L16,
- une première et une deuxième électrovannes de conditionnement 6a, 6b pilotables par l'unité de séquençage 4, agencées en dérivation de l'entrée 7 de la pompe de conditionnement 3, et
- une première et une deuxième électrovannes de mesure 8a, 8b pilotables par l'unité de séquençage 4, agencées en dérivation de l'entrée 9 du au moins un analyseur de gaz 2.

Plusieurs analyseurs de gaz 2, pour la mesure de concentrations de différents gaz ou groupe d'espèces gazeuses, peuvent être raccordés à l'entrée 9 pour prélever simultanément dans une même ligne de prélèvement.

La première électrovanne de conditionnement 6a et la première électrovanne de mesure 8a sont raccordées à des électrovannes de prélèvement 5 d'une première série S1 de lignes de prélèvement L1, L3, L5, L7, L9, L11, L13, L15. La première série S1 de lignes de prélèvement comporte au moins une ligne de prélèvement.

La deuxième électrovanne de conditionnement 6b et la deuxième électrovanne de mesure 8b sont raccordées à des électrovannes de prélèvement 5 d'une deuxième série S2 de lignes de prélèvement L2, L4, L6, L8, L10, L12, L14, L16. La deuxième série S2 de lignes de prélèvement comporte au moins une ligne de prélèvement.

Il y a par exemple au moins cinq lignes de prélèvements L1-L16 chacune munie d'une électrovanne de prélèvement 5, tel que seize lignes de prélèvements L1-16. La première série S1 de lignes de prélèvement comporte par exemple un même nombre de lignes de prélèvement que la deuxième série S2 (huit dans l'exemple).

Le programme de séquençage définit par exemple un ordre des mesures à réaliser qui alterne les mesures à réaliser dans chaque série S1, S2 de lignes de prélèvement. Cette solution permet de limiter le nombre de vannes utilisées, ce qui permet de simplifier le dispositif et maitriser les coûts.

En fonctionnement, on conditionne une ligne de prélèvement L1-L16, par exemple une seule et unique ligne de prélèvement, dont la mesure est programmée consécutivement à une ligne de prélèvement L1-L16 en cours de mesure pendant que l'on mesure dans la ligne de prélèvement L1-L16 avec le au moins un analyseur de gaz 2. On alterne par exemple les mesures à réaliser dans chacune des deux séries S1, S2 de lignes de prélèvement.

En prenant pour exemple un programme de séquençage définissant qu'une mesure de concentration des espèces gazeuses doit être réalisée dans la ligne de prélèvement L5 de la première série S1 suivie d'une mesure dans la ligne de prélèvement L4 de la deuxième série S2, on conditionne la ligne de prélèvement L4 de la deuxième série S2 pendant que l'on mesure dans la ligne de prélèvement L5 avec le au moins un analyseur de gaz 2.

Pour cela, l'unité de séquençage 4 pilote la mise en communication de la ligne de prélèvement L4 avec la pompe de conditionnement 3 en ouvrant l'électrovanne de prélèvement 5 de la ligne de prélèvement L4 ainsi que la deuxième électrovanne de conditionnement 6b, la première électrovanne de conditionnement 6a raccordée aux électrovannes de prélèvement 5 de la première série S1 étant fermée.

Simultanément, l'unité de séquençage 4 pilote la mise en communication de la ligne de prélèvement L5 avec le au moins un analyseur de gaz 2 en ouvrant l'électrovanne de prélèvement 5 de la ligne de prélèvement L5 ainsi que la première électrovanne de mesure 8a, la deuxième électrovanne de mesure 8b raccordée aux électrovannes de prélèvement 5 de la deuxième série S2 étant fermée (étape 101, Figure 2).

Puis, après avoir réalisé la mesure dans la ligne de prélèvement L5, l'unité de séquençage 4 pilote la mise en communication de la ligne de prélèvement L4 avec le au moins un analyseur de gaz 2 en ouvrant l'électrovanne de prélèvement 5 de la ligne de prélèvement L4 ainsi que la deuxième électrovanne de mesure 8b, la première électrovanne de mesure 8a raccordée aux électrovannes de prélèvement 5 de la première série S1 étant fermée.

Simultanément, l'unité de séquençage 4 pilote la mise en communication de la ligne de prélèvement de la première série S1 dont une mesure de concentration des espèces gazeuses doit être réalisée consécutivement, avec la pompe de conditionnement 3 (étape 102, Figure 2).

Les opérations peuvent se poursuivre ainsi jusqu'à ce que toutes les mesures définies dans le programme de séquençage soient réalisées dans l'ordre. Ces opérations peuvent être réitérées en boucle.

Il est ainsi possible de mesurer la concentration de différentes espèces gazeuses véhiculées par l'air à différents endroits de la salle blanche avec un même ensemble d'analyseurs de gaz 2, la station de mesure 1 adressant un port d'entrée dans chaque zone de test de la salle blanche. On limite ainsi les coûts.

Les lignes de prélèvement peuvent donc être conditionnées dans des conditions de pompage optimales et rapides.

En effet, le temps de mesure est réduit car le conditionnement de la ligne de prélèvement est réalisé en temps masqué.

En outre, un maximum de pompage peut être utilisé pour le conditionnement et ce, quelque soit le nombre de lignes de prélèvement. Le nombre de lignes de prélèvement peut donc être augmenté sans que cela modifie les performances de pompage pour le conditionnement. Le gaz ainsi aspiré avant que la ligne soit mesurée permet un dégazage de la ligne de prélèvement. Les mesures peuvent alors être plus précises, en particulier dans les cas où un changement de concentration important a lieu entre deux zones à tester consécutivement et où un temps de dégazage suffisant est nécessaire pour obtenir une mesure réellement représentative de la concentration dans la zone de test.

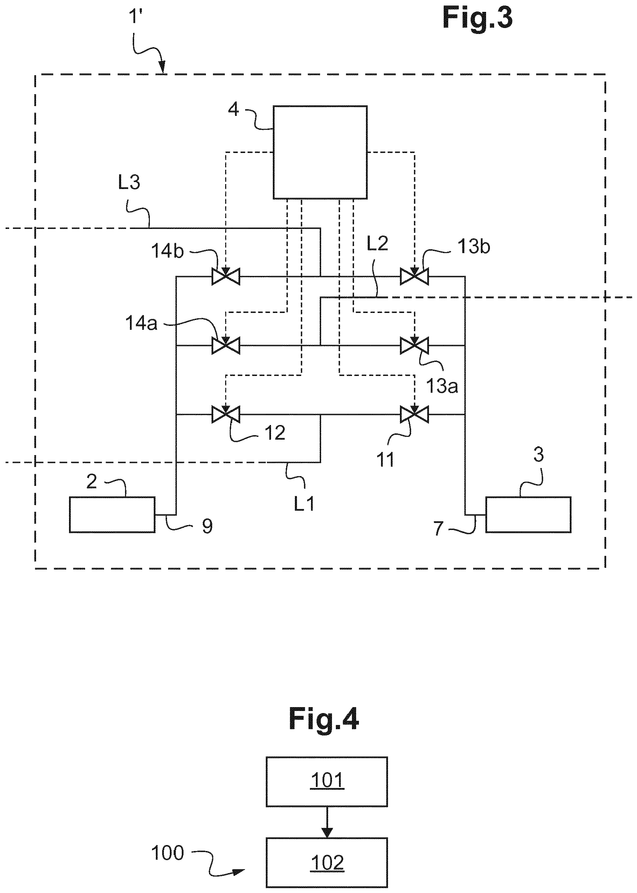

La Figure 3 illustre un deuxième exemple de réalisation de la station de mesure 1'.

Dans cet exemple, la station de mesure 1' comporte :
- une première électrovanne de conditionnement 11 pilotable par l'unité de séquençage 4, agencée à l'entrée 7 de la pompe de conditionnement 3,
- une première électrovanne de mesure 12 pilotable par l'unité de séquençage 4, agencée à l'entrée 9 du au moins un analyseur de gaz 2,
- au moins une deuxième électrovanne de conditionnement 13a, 13b pilotable par l'unité de séquençage 4, agencée en dérivation de la première électrovanne de conditionnement 11 et de l'entrée 7 de la pompe de conditionnement 3, et
- au moins une deuxième électrovanne de mesure 14a, 14b pilotable par l'unité de séquençage 4 agencée en dérivation de la première électrovanne de mesure 12 et de l'entrée 9 du au moins un analyseur de gaz 2.

La première électrovanne de mesure 12 et la première électrovanne de conditionnement 11 sont raccordées à une ligne de prélèvement L1.

La au moins deuxième électrovanne de mesure 14a, 14b et la au moins deuxième électrovanne de conditionnement 13a, 13b sont raccordées à au moins une ligne de prélèvement L2, L3.

Il y a dans l'exemple illustré, trois lignes de prélèvement L1-L3, c'est à dire deux deuxièmes électrovannes de conditionnement 13a, 13b agencées en dérivation de la première électrovanne de conditionnement 11 et de l'entrée 7 de la pompe de conditionnement 3, et deux deuxièmes électrovannes de mesure 14a, 14b agencées en dérivation de la première électrovanne de mesure 12 et de l'entrée 9 du au moins un analyseur de gaz 2.

Les deux deuxièmes électrovannes de mesure 14a, 14b et les deux deuxièmes électrovannes de conditionnement 13a, 13b sont raccordées à deux lignes de prélèvement L2, L3 respectives.

On prévoit par exemple que la station de mesure 1' selon ce deuxième mode de réalisation comporte moins de quatre deuxièmes électrovannes de conditionnement et moins de quatre deuxièmes électrovannes de mesure, la station de mesure 1 étant reliée à au plus cinq lignes de prélèvement L1-L5. En effet, au-delà de cinq, le nombre d'électrovannes nécessaires augmente le coût de la station de mesure 1' de façon relativement importante.

En fonctionnement, on conditionne une ligne de prélèvement, par exemple une seule et unique ligne de prélèvement, dont la mesure est programmée consécutivement à une ligne de prélèvement en cours de mesure pendant que l'on mesure dans la ligne de prélèvement avec le au moins un analyseur de gaz 2.

En prenant comme exemple un programme de séquençage définissant qu'une mesure de concentration des espèces gazeuses doit être réalisée dans la ligne de prélèvement L2 suivie d'une mesure dans la ligne de prélèvement L3, on conditionne la ligne de prélèvement L3 pendant que l'on mesure dans la ligne de prélèvement L2 avec le au moins un analyseur de gaz 2.

Pour cela, l'unité de séquençage 4 pilote la mise en communication de la ligne de prélèvement L3 avec la pompe de conditionnement 3 en ouvrant la deuxième électrovanne de prélèvement 13b de la ligne de prélèvement L3, la deuxième électrovanne de mesure 14b de la ligne de prélèvement L3 étant fermée.

Simultanément, l'unité de séquençage 4 pilote la mise en communication de la ligne de prélèvement L2 avec le au moins un analyseur de gaz 2 en ouvrant la deuxième électrovanne de mesure 14a de la ligne de prélèvement L2, la deuxième électrovanne de prélèvement 13a étant fermée (étape 101, Figure 4).

Puis, après avoir réalisé la mesure dans la ligne de prélèvement L2, l'unité de séquençage 4 pilote la mise en communication de la ligne de prélèvement L3 avec le au moins un analyseur de gaz 2 en ouvrant la deuxième électrovanne de mesure 14b de la ligne de prélèvement L3 et en fermant la deuxième électrovanne de prélèvement 13b de la ligne de prélèvement L3 et la deuxième électrovanne de mesure 14a de la ligne de prélèvement L2.

Simultanément, l'unité de séquençage 4 pilote la mise en communication de la ligne de prélèvement dont une mesure de concentration des espèces gazeuses doit être réalisée consécutivement, avec la pompe de conditionnement 3 (étape 102, Figure 4).

Les opérations peuvent se poursuivre ainsi jusqu'à ce que toutes les mesures définies dans le programme de séquençage soient réalisées dans l'ordre. Ces opérations peuvent être réitérées en boucle.

## Revendications

1. Station de mesure (1 ; 1') de la contamination moléculaire véhiculée par l'air comportant au moins un analyseur de gaz (2) et une pompe de conditionnement (3) comportant en outre une unité de séquençage (4) comportant un programme de séquençage définissant un ordre des mesures à réaliser pour au moins deux lignes de prélèvement (L1-L16), l'unité de séquençage (4) étant configurée pour piloter la mise en communication d'une ligne de prélèvement (L1-L16) à mesurer dont la mesure est programmée consécutivement à une ligne de prélèvement (L1-L16) en cours de mesure, avec la pompe de conditionnement (3) pendant le pilotage de la mise en communication de la ligne de prélèvement (L1-L16) en cours de mesure avec le au moins un analyseur de gaz (2), la station de mesure (1) **caractérisée en ce qu'**elle comporte:
- une électrovanne de prélèvement (5) pilotable par l'unité de séquençage (4) sur chaque ligne de prélèvement (L1-L16),
- une première et une deuxième électrovannes de conditionnement (6a, 6b) pilotables par l'unité de séquençage (4), agencées en dérivation de l'entrée (7) de la pompe de conditionnement (3),
- une première et une deuxième électrovannes de mesure (8a, 8b) pilotables par l'unité de séquençage(4), agencées en dérivation de l'entrée (9) du au moins un analyseur de gaz (2), la première électrovanne de conditionnement (6a) et la première électrovanne de mesure (8a) étant raccordées à des électrovannes de prélèvement (5) d'une première série (S1) de lignes de prélèvement (L1, L3, L5, L7, L9, L11, L13, L15), la deuxième électrovanne de conditionnement (6b) et la deuxième électrovanne de mesure (8b) étant raccordées à des électrovannes de prélèvement (5) d'une deuxième série (S2) de lignes de prélèvement (L2, L4, L6, L8, L10, L12, L14, L16).

2. Station de mesure (1) selon la revendication précédente, **caractérisée** en que l'unité de séquençage (4) est configurée pour piloter la mise en communication d'une seule et unique ligne de prélèvement (L1-L16) à mesurer dont la mesure est programmée consécutivement à une ligne de prélèvement (L1-L16) en cours de mesure, avec la pompe de conditionnement (3) pendant le pilotage de la mise en communication de la ligne de prélèvement (L1-L16) en cours de mesure avec le au moins un analyseur de gaz (2).

3. Station de mesure (1) selon l'une des revendications précédentes, **caractérisée en ce qu'**elle comporte au moins cinq électrovannes de prélèvement (5).

4. Station de mesure (1) selon l'une des revendications précédentes, **caractérisée en ce que** la première série (S1) de lignes de prélèvement comporte un même nombre de lignes de prélèvement que la deuxième série (S2).

5. Station de mesure (1) selon l'une des revendications précédentes, **caractérisée en ce que** le programme de séquençage définit un ordre des mesures à réaliser alternant les mesures à réaliser dans chaque série (S1, S2) de lignes de prélèvement.

6. Procédé de mesure (100) de la contamination moléculaire véhiculée par l'air au moyen d'une station de mesure (1 ; 1') selon l'une des revendications précédentes, **caractérisé en ce qu'**on conditionne une ligne de prélèvement (L1-L16) dont la mesure est programmée consécutivement à une ligne de prélèvement (L1- L16) en cours de mesure pendant que l'on mesure dans la ligne de prélèvement (L1- L16) avec au moins un analyseur de gaz (2).

7. Procédé de mesure (100) selon la revendication précédente, **caractérisé en ce qu'**on alterne les mesures à réaliser dans chacune des deux séries (S1, S2) de lignes de prélèvement, une première série (S1) de lignes de prélèvement (L1, L3, L5, L7, L9, L11, L13, L15) étant raccordée à une première électrovanne de conditionnement (6a) et à une première électrovanne de mesure (8a), une deuxième série (S2) de lignes de prélèvement (L2, L4, L6, L8, L10, L12, L14, L16) étant raccordée à une deuxième électrovanne de conditionnement (6b) et à une deuxième électrovanne de mesure (8b).

8. Procédé de mesure (100) selon l'une des revendications 6 ou 7, **caractérisé en ce que** la ligne de prélèvement (L1-L16) conditionnée pendant que l'on mesure dans la ligne de prélèvement (L1- L16) avec au moins un analyseur de gaz (2) est unique.

## Patentansprüche

1. Messstation (1; 1') zum Messen der luftgetragenen molekularen Verunreinigung, umfassend mindestens einen Gasanalysator (2) und eine Konditionierpumpe (3), umfassend ferner eine Sequenzierungseinheit (4), die ein Sequenzierungsprogramm umfasst, das eine Serienfolge der auszuführenden Messungen für mindestens zwei Entnahmeleitungen (L1-L16) definiert, wobei die Sequenzierungseinheit (4) dazu konfiguriert ist, das Herstellen der Verbindung einer zu messenden Entnahmeleitung (L1-L16), deren Messung folgend auf eine gerade gemessene Entnahmeleitung (L1-L16) programmiert ist, mit der Konditionierpumpe (3) während der Ansteuerung des Herstellens der Verbindung der gerade gemessenen Entnahmeleitung (L1-L16) mit dem mindestens einen Gasanalysator (2) anzusteuern, wobei die Messstation (1) **dadurch gekennzeichnet ist, dass** sie umfasst
- ein Entnahmemagnetventil (5), das von der Sequenzierungseinheit (4) ansteuerbar ist, an jeder Entnahmeleitung (L1-L16),
- ein erstes und ein zweites Konditioniermagnetventil (6a, 6b), die von der Sequenzierungseinheit (4) ansteuerbar sind und vom Einlass (7) der Konditionierpumpe (3) abzweigend angeordnet sind,
- ein erstes und ein zweites Messmagnetventil (8a, 8b), die von der Sequenzierungseinheit (4) ansteuerbar sind und vom Einlass (9) des mindestens einen Gasanalysators (2) abzweigend angeordnet sind, wobei das erste Konditioniermagnetventil (6a) und das erste Messmagnetventil (8a) mit Entnahmemagnetventilen (5) einer ersten Serie (S1) von Entnahmeleitungen (L1, L3, L5, L7, L9, L11, L13, L15) verbunden sind, wobei das zweite Konditioniermagnetventil (6b) und das zweite Messmagnetventil (8b) mit Entnahmemagnetventilen (5) einer zweiten Serie (S2) von Entnahmeleitungen (L2, L4, L6, L8, L10, L12, L14, L16) verbunden sind.

2. Messstation (1) nach dem vorhergehenden Anspruch, **dadurch gekennzeichnet, dass** die Sequenzierungseinheit (4) dazu konfiguriert ist, das Herstellen der Verbindung einer einzigen zu messenden Entnahmeleitung (L1-L16), deren Messung folgend auf eine gerade gemessene Entnahmeleitung (L1-L16) programmiert ist, mit der Konditionierpumpe (3) während der Ansteuerung des Herstellens der Verbindung der gerade gemessenen Entnahmeleitung (L1-L16) mit dem mindestens einen Gasanalysator (2) anzusteuern.

3. Messstation (1) nach einem der vorhergehenden Ansprüche, **dadurch gekennzeichnet, dass** sie mindestens fünf Entnahmemagnetventile (5) umfasst.

4. Messstation (1) nach einem der vorhergehenden Ansprüche, **dadurch gekennzeichnet, dass** die erste Serie (S1) von Entnahmeleitungen die gleiche Anzahl von Entnahmeleitungen wie die zweite Serie (S2) umfasst.

5. Messstation (1) nach einem der vorhergehenden Ansprüche, **dadurch gekennzeichnet, dass** das Sequenzierungsprogramm eine Reihenfolge der auszuführenden Messungen definiert, bei der die auszuführenden Messungen in jeder Serie (S1, S2) von Entnahmeleitungen abwechseln.

6. Messverfahren (100) zum Messen der luftgetragenen molekularen Verunreinigung mit einer Messstation (1; 1') nach einem der vorhergehenden Ansprüche, **dadurch gekennzeichnet, dass** man eine Entnahmeleitung (L1-L16), deren Messung folgend auf eine gerade gemessene Entnahmeleitung (L1-L16) programmiert ist, konditioniert, während man in der Entnahmeleitung (L1-L16) mit mindestens einem Gasanalysator (2) misst.

7. Messverfahren (100) nach dem vorhergehenden Anspruch, **dadurch gekennzeichnet, dass** man die auszuführenden Messungen in jeder der beiden Serien (S1, S2) von Entnahmeleitungen abwechselt, wobei eine erste Serie (S1) von Entnahmeleitungen (L1, L3, L5, L7, L9, L11, L13, L15) mit einem ersten Konditioniermagnetventil (6a) und mit einem ersten Messmagnetventil (8a) verbunden ist, wobei eine zweite Serie (S2) von Entnahmeleitungen (L2, L4, L6, L8, L10, L12, L14, L16) mit einem zweiten Konditioniermagnetventil (6b) und mit einem zweiten Messmagnetventil (8b) verbunden ist.

8. Messverfahren (100) nach einem der Ansprüche 6 oder 7, **dadurch gekennzeichnet, dass** die Entnahmeleitung (L1-L16), die konditioniert wird, während man in der Entnahmeleitung (L1-L16) mit mindestens einem Gasanalysator (2) misst, eine einzige ist.

## Claims

1. Station (1; 1') for measuring airborne molecular contamination comprising at least one gas analyser (2) and a conditioning pump (3) further comprising a sequencing unit (4) comprising a sequencing program defining an order of the measurements to be performed for at least two sampling lines (L1-L16), the sequencing unit (4) being configured to control the connecting of a sampling line (L1-L16) to be measured for which the measurement is programmed to follow a sampling line (L1-L16) currently being measured, with the conditioning pump (3) during the controlling of the connecting of the sampling line (L1-L16) currently being measured with the at least one gas analyser (2), the measurement station (1) being **characterized in that** it comprises:
- a sampling electrovalve (5) that can be controlled by the sequencing unit (4) on each sampling line (L1-L16),
- a first and a second conditioning electrovalve (6a, 6b) that can be controlled by the sequencing unit (4), arranged as a branch of the input (7) of the conditioning pump (3),
- a first and a second measurement electrovalve (8a, 8b) that can be controlled by the sequencing unit (4), arranged as a branch of the input (9) of the at least one gas analyser (2), the first conditioning electrovalve (6a) and the first measurement electrovalve (8a) being connected to sampling electrovalves (5) of a first series (S1) of sampling lines (L1, L3, L5, L7, L9, L11, L13, L15), the second conditioning electrovalve (6b) and the second measurement electrovalve (8b) being connected to sampling electrovalves (5) of a second series (S2) of sampling lines (L2, L4, L6, L8, L10, L12, L14, L16).

2. Measurement station (1) according to the preceding claim, **characterized in that** the sequencing unit (4) is configured to control the connecting of a single sampling line (L1-L16) to be measured for which the measurement is programmed following a sampling line (L1-L16) currently being measured, with the conditioning pump (3) during the controlling of the connecting of the sampling line (L1-L16) currently being measured with the at least one gas analyser (2).

3. Measurement station (1) according to one of the preceding claims, **characterized in that** it comprises at least five sampling electrovalves (5) .

4. Measurement station (1) according to one of the preceding claims, **characterized in that** the first series (S1) of sampling lines comprises a same number of sampling lines as the second series (S2) .

5. Measurement station (1) according to one of the preceding claims, **characterized in that** the sequencing program defines an order of the measurements to be performed alternating the measurements to be performed in each series (S1, S2) of sampling lines.

6. Method for measuring (100) airborne molecular contamination by means of a measurement station (1; 1') according to one of the preceding claims, **characterized in that** a sampling line (L1-L16) for which the measurement is programmed following a sampling line (L1-L16) currently being measured is conditioned while measuring in the sampling line (L1-L16) with at least one gas analyser (2).

7. Measurement method (100) according to the preceding claim, **characterized in that** the measurements to be performed in each of the two series (S1, S2) of sampling lines are alternated, a first series (S1) of sampling lines (L1, L3, L5, L7, L9, L11, L13, L15) being connected to a first conditioning electrovalve (6a) and to a first measurement electrovalve (8a), a second series (S2) of sampling lines (L2, L4, L6, L8, L10, L12, L14, L16) being connected to a second conditioning electrovalve (6b) and to a second measurement electrovalve (8b).

8. Measurement method (100) according to one of Claims 6 and 7, **characterized in that** the sampling line (L1-L16) conditioned while measuring in the sampling line (L1-L16) with the at least one gas analyser (2) is singular.
